# EUROPEAN PATENT APPLICATION

(11) **EP 2 031 011 A1**
(43) Date of publication of application: **04.03.2009**
(21) Application number: 07744407.3
(22) Date of filing: 30.05.2007
(51) Int. Cl.: C08H 1/06, A01N 59/06, A01N 61/00, A01P 3/00, B01J 20/26, C08B 11/12, C08F 8/44, C08H 5/00, C08J 3/12, C08L 1/26, C08L 29/04, C08L 89/04, C08L 101/00

(54) **RESIN POWDER CONTAINING ALUMINUM SALT, PROCESS FOR PRODUCTION OF THE SAME, AND RESIN COMPOSITION, PHOSPHOROUS ADSORBENT, ANTIBACTERIAL AGENT OR ANTIFUNGAL AGENT COMPRISING THE SAME**

(30) Priority: 02.06.2006 JP 2006155365; 06.07.2006 JP 2006186244; 05.03.2007 JP 2007054689; 05.03.2007 JP 2007054690
(71) Applicant: Kaneka Corporation, Kita-ku Osaka-shi, Osaka 530-8288 (JP)
(72) Inventor: YAMASHITA, Kenji c/o Kaneka Corporation, Osaka 566-0072 (JP)
(74) Representative: Vossius & Partner
(86) International application number: PCT/JP2007/060994
(87) International publication number: WO 2007/142097

(57) **Abstract**

An aluminum salt-containing resin powder of the present invention includes at lest one matrix resin component selected from regenerated collagen, polyvinyl alcohol and carboxymethyl cellulose; and an aluminum salt. The aluminum salt is chemically bonded to the matrix resin component, and the resultant is powdered. A resin composition of the present invention includes 0.1 wt% or more and 80 wt% or less of the aluminum salt-containing resin powder and 20 wt% or more and 99.9 wt% or less of a resin other than the aluminum salt-containing resin. Thus, the present invention provides an aluminum salt-containing resin powder having a high phosphorus adsorption property, a high antibacterial property and a high antifungal property, and a resin composition containing the aluminum salt-containing resin powder.

## Description

### Technical Field

The present invention relates to an aluminum salt-containing resin powder having a high phosphorus adsorption property, a high antibacterial property and a high antifungal property, a process for producing the same, a resin composition containing the same, and applications thereof.

### Background Art

Eutrophication due to an increase of phosphorus in lakes, reservoirs, rivers, and household drainage such as wastewater from households causes harmful effects on fish farming such as occurrence of red tide, etc., and various other harmful effects on the environment are of great concern.

The use of aluminum salt, such as aluminum sulfate and ferric chloride, as well as iron salt and lime as a phosphorus removing substance is known conventionally. However, there are problems in terms of cost and technical aspects, and therefore they have not yet come into practical use. Studies to address the problems have been made, and it has been proposed that volcanic ash soils and weathered products of pumice (hereinafter referred to as "allophane") can be used as a phosphorus adsorption/removing agent (Patent Documents 1 to 2), which was discovered following observation of a phosphorus fixation phenomenon in which phosphorus contained in a fertilizer applied to a soil made of volcanic ash or a weathered product of pumice is adsorbed strongly to the soil. However, allophane also has problems in terms of cost and technical aspects, and thus has not yet come into practical use as an agent for removing phosphorus contained in lakes and household drainage.

Many microorganisms exist in the environment in which humans live. Particularly, Japan where it is hot and humid is in an environment in which prokaryotes such as bacteria, eukaryotes such as mold and yeast, as well as moss and algae, easily can reproduce and increase in number. Accordingly, products that come in frequent contact with people may serve as a source of infection for pathogens and harmful bacteria, and thus such products are required to have an antibacterial property from the viewpoint of safety and hygiene in daily life. For this reason, conventionally, products in which an antibacterial agent or antifungal agent is blended into a resin material are produced (Patent Documents 6 to 7). It is desirable to suppress the blending amount of the antibacterial agent or antifungal agent in the resin material to the lowest level possible considering the production cost and the like, but no other means to impart an antibacterial property or antifungal property to products is known conventionally other than the use of an antibacterial agent or antifungal agent. In addition, conventional resin articles produced using a resin composition obtained by adding an antibacterial agent or antifungal agent to a resin material are problematic in that the dispersibility of the antibacterial agent or antifungal agent easily is affected by the atmosphere at the time of adding, and as a result, the antibacterial ability or antifungal ability varies and is not stable.

Another proposal also has been made in which a natural product-derived polymer gel such as alginic acid soda or chitosan is immobilized in a polyurethane foam having 8 to 13 pores per inch (25 mm), and phosphate ions are adsorbed (Patent Document 8). However, this proposal is also problematic in terms of phosphate ion adsorption performance, and thus it has not come into practical use as an agent for removing phosphorus contained in lakes and household drainage.
Patent Document 1: JP S58-177195A
Patent Document 2: Japanese Patent No. 3011213
Patent Document 3: JP 2000-202953A
Patent Document 4: JP 2001-254281A
Patent Document 5: JP 2005-200612A
Patent Document 6: JP 2005-297661A
Patent Document 7: JP 2006-102000A
Patent Document 8: JP H7-39754A

### Disclosure of Invention

### Problem to be Solved by the Invention

In order to improve the problems encountered in conventional technology, the present invention provides an aluminum salt-containing resin powder having a high phosphorus adsorption property, a high antibacterial property and a high antifungal property, a process for producing the same, and a resin composition, a phosphorus adsorbent, an antibacterial agent and an antifungal agent that contain the same.

### Means for Solving Problem

An aluminum salt-containing resin powder according to the present invention includes: at least one matrix resin component selected from regenerated collagen, polyvinyl alcohol and carboxymethyl cellulose; and an aluminum salt, and the aluminum salt is bonded chemically to the matrix resin component, and the resultant is powdered.

A resin composition according to the present invention includes 0.1 wt% or more and 80 wt% or less of the aluminum salt-containing resin powder and 20 wt% or more and 99.9 wt% or less of a resin other than the aluminum salt-containing resin.

A process for producing an aluminum salt-containing resin powder according to the present invention includes the steps of: bringing an aluminum salt into contact with at least one water-soluble matrix resin gel component selected from regenerated collagen, polyvinyl alcohol and carboxymethyl cellulose so as to bond the aluminum salt chemically to the matrix resin gel component to obtain a water insoluble resin; drying the water-insoluble resin; and pulverizing the dried water insoluble resin into powder.

A phosphorus adsorbent according to the present invention includes the aluminum salt-containing resin powder or the resin composition.

An antibacterial agent according to the present invention includes the aluminum salt-containing resin powder or the resin composition.

An antifungal agent according to the present invention includes the aluminum salt-containing resin powder or the resin composition.

### Best Mode for Carrying Out the Invention

The aluminum salt-containing resin powder of the present invention has a high phosphorus adsorption capability, and therefore is capable of adsorbing elemental phosphorus or a phosphorus compound contained in lakes, reservoirs, rivers or household drainage.

Because the aluminum salt-containing resin powder of the present invention has a high phosphorus adsorption capability, it traps phosphorus, which is a nutrient for bacteria, and exhibits an antibacterial property. Also, the aluminum salt-containing resin powder is superior in terms of antibacterial property and resin dispersibility, and thus by blending it with, for example, a resin composition, the antibacterial property can be imparted to various articles such as interior materials for vehicles, aircraft, ships, etc., outdoor materials for furniture, etc., cushion materials, clothing materials, wrapping materials, tableware materials, stationery, filters, and components of electrical appliances (e.g., personal computers, cell phones).

Furthermore, because the aluminum salt-containing resin powder of the present invention has a high phosphorus adsorption capability, it traps phosphorus, which is a nutrient for bacteria, and exhibits an antifungal property. Also, the aluminum salt-containing resin powder is superior in terms of antifungal property and resin dispersibility, and thus by blending it with, for example, a resin composition, the antifungal property can be imparted to various articles such as interior materials for vehicles, aircraft, ships, etc., outdoor materials for furniture, etc., cushion materials, clothing materials, wrapping materials, tableware materials, stationery, filters, and components of electrical appliances (e.g., personal computers, cell phones).

The aluminum-containing resin powder of the present invention can be used, by mixing with an aqueous medium, as a spray agent.

A collagen powder of the present invention will be described below. The present invention can provide a new collagen powder that can solve the quality problem of conventional collagen powders by producing a solubilized collagen solution from the skin, bones and tendons of animals such as bovines, pigs, horses, deer, rabbits, birds and fish, and subjecting it to a cross-linking treatment. Furthermore, the solubilized collagen solution is spun into a regenerated collagen fiber, and thereby, thorough purification of collagen is possible, and dense cross-linkage is performed in a fibrillation step through spinning, and thereby it is possible to provide a completely new collagen powder.

As a process for producing the above regenerated collagen, for example, as disclosed in JP 2002-249982A, it is preferable to use a split hide portion as a raw material. Split hide is obtained from fresh split hide or salted rawhide obtained from animals such as bovines, pigs, horses, deer, rabbits, birds and fish. Such split hide mostly is made of insoluble collagen fibers, and is used after a flesh portion normally attached in the form of a net and a salt component used for preventing corrosion and alteration has been removed. Other materials, such as the bones and tendons of the above-listed animals, can be used as well.

In the insoluble collagen fibers, impurities exist such as lipids such as glyceride, phospholipid and unesterified fatty acid; and proteins other than collagen such as glycoprotein and albumin. These impurities significantly affect the quality including luster and strength, the odor, etc. when powdering. Accordingly, it is preferable to remove these impurities in advance by, for example, subjecting it to liming so as to hydrolyze the fat components contained in the insoluble collagen fibers to disentangle the collagen fibers, and followed by ordinary leather processing such as an acid/alkali treatment, an enzyme treatment, and a solvent treatment.

The insoluble collagen processed as described above is subjected to a solubilization process in order to dissociate the cross-linked peptide portion. As a method for the solubilization process, a commonly used and known alkali solubilization method, enzyme solubilization method, etc., can be used. When using the alkali solubilization method, it is preferable to neutralize with, for example, an acid such as hydrochloric acid. It is also possible to use the method disclosed in JP S46-15033B, which is conventionally known as an improved method of alkali solubilization method.

The enzyme solubilization method is advantageous in that regenerated collagen of uniform molecular weight can be obtained, and it can be used -preferably in the present invention. As the enzyme solubilization method, for example, the methods described in JP S43-25829B, JP S43-27513B, etc. can be used. Also, a combined use of the alkali solubilization method and the enzyme solubilization method is possible.

When the collagen having undergone the solubilization process described above is subjected to further operations, such as pH adjustment, salting-out, washing with water and a solvent treatment, it is possible to obtain regenerated collagen superior in quality and the like. Accordingly, it is preferable to subject it to these treatments. The obtained solubilized collagen is dissolved using an acid solution adjusted to pH 2 to 4.5 with an acid such as hydrochloric acid, acetic acid or lactic acid such that a raw material solution having a predetermined concentration of, for example, approximately 1 to 15 wt%, and preferably approximately 2 to 10 wt% can be obtained. The obtained aqueous collagen solution may be deaerated under a reduced pressure and agitation as appropriate, and filtered to remove small unwanted matter, or components insoluble in water. The obtained aqueous solution of solubilized collagen further may be blended with appropriate amounts of additives, such as a stabilizer and a water-soluble polymer compound, as appropriate according to the purpose, for example, increasing mechanical strength, improving water/heat resistance, enhancing luster, improving spinning property, preventing coloring, preventing corrosion, etc.

The aqueous solution of solubilized collagen is passed through, for example, a spinning nozzle or slit, and discharged to an aqueous solution of inorganic salt to form regenerated collagen. As the aqueous solution of inorganic salt, an aqueous solution containing a water-soluble inorganic salt such as, for example, sodium sulfate, sodium chloride or ammonium sulfate can be used. Usually, the concentration of the inorganic salt is adjusted to 10 to 40 wt%. It is preferable to adjust the pH of the aqueous solution of inorganic salt to, usually, pH 2 to 13, and preferably pH 4 to 12, by blending in, for example, a metal salt, such as sodium borate or sodium acetate, hydrochloric acid, boric acid, acetic acid, sodium hydroxide, etc. When the pH falls within this range, a desired collagen powder in which the peptide bond of the collagen is not readily hydrolyzed can be obtained. There is no particular limitation on the temperature of the aqueous solution of inorganic salt, but preferably, the temperature is usually 35°C or lower. When the temperature is 35°C or lower, the soluble collagen does not alter, and thus a high strength can be maintained, and stable production is possible. There is no particular limitation on the lower limit of the temperature, but usually, the lower limit can be adjusted as appropriate according to the solubility of the inorganic salt.

The free amino groups of the collagen are modified with an alkyl group having a hydroxyl group or alkoxy group at the β position or the γ position and a carbon number main chain of 2 to 20. As used herein, "carbon number main chain" refers to a continuous carbon chain of an alkyl group bonded to an amino group, and the number of carbon atoms that are present with another atom interposed therebetween is not taken into account. As the reaction that modifies free amino groups, commonly known amino group alkylation reaction can be used. Considering reactivity, ease of processing after reaction, etc, the alkyl group having a hydroxyl group or alkoxy group at the β position and a carbon number of 2 to 20 preferably is a compound represented by the following general formula (2).

-CH₂-CH(OX)-R (2)

where R represents a substituent represented by R¹-, R²-O-CH₂- or R²-COO-CH₂-; R¹ in the substituent is a hydrocarbon group having a carbon number of 2 or more, or CH₂Cl; R² represents a hydrocarbon group having a carbon number of 4 or more; and X represents hydrogen or a hydrocarbon group).
Preferred examples of the general formula (2) include glycidyl group, 1-chloro-2-hydroxypropyl group, and 1,2-dihydroxy propyl group. In addition, other possibilities include a structure in which a glycidyl group is added to free amino groups of collagen, and a structure in which an epoxy compound used is ring-opening added or ring-opening polymerized, starting from the hydroxyl group included in the alkyl group described as a preferred group above, and the alkyl group described above is incorporated as an end structure of the resultant obtained by the addition and/or polymerization.

The amino acids that constitute free amino groups of the regenerated collagen are lysine, hydroxylysine and the like. As an amino acid originally constituting collagen, arginine is present, but when hydrolysis is performed in an alkaline condition in order to obtain regenerated collagen described above, the amino groups of ornithine produced as a result of partial hydrolysis also are alkylated. In addition, the reaction proceeds also due to secondary amine contained in histidine.

The free amino group modification ratio can be measured by amino acid analysis, and is calculated with respect to an amino acid analysis value of regenerated collagen fibers before the alkylation reaction or a known composition of free amino acid constituting collagen used as a raw material. In the amino group modification of the present invention, it is sufficient that the structure modified by an alkyl group having a hydroxyl group or alkoxy group at the β position or the γ position and a carbon number of 2 or more accounts for 50% or more of the free amino groups, and other portions may be free amino groups or a structure modified by another substituent. The free amino acid modification ratio of the regenerated collagen needs to be 50% or more, more preferably 65% or more, and even more preferably 80% or more. When the reaction ratio is low, favorable characteristics in terms of heat resistance cannot be obtained.

In the free amino group modification, usually, one molecule of an alkylating agent reacts per one free amino group. It is of course possible that two or more molecules react. It is also possible that intramolecular or intermolecular cross-linking reaction may be present in the hydroxyl group or alkoxy group present at the β position or the γ position of the alkyl group bonded to free amino groups via other functional groups. Specific examples of alkylation reaction include, but are not limited to, addition reaction of an epoxy compound, addition reaction of an aldehyde compound that has a hydroxyl group or its derivative at the α position or the β position, and subsequent reduction reaction, and substitution reaction of a halide, alcohol, amine, etc. having a hydroxyl group or alkoxy group at the 6 position or the γ position and a carbon number of 2 or more.

Examples of the organic compound that can be used as an alkylating agent in the present invention include aldehydes, epoxies and phenol derivatives. Among them, an epoxy compound is preferable because the modification reaction with the epoxy compound exhibits superior characteristics because of reactivity, and ease of treatment conditions. Particularly, a mono-functional epoxy compound is preferable.

Specific examples of the mono-functional epoxy compound that can be used here include, but are not limited to: olefin oxides such as ethylene oxide, propylene oxide, butylene oxide, isobutylene oxide, octene oxide, styrene oxide, methyl styrene oxide, epichlorohydrin, epibromohydrin, and glycidol; glycidyl ethers such as glycidyl methyl ether, butyl glycidyl ether, octyl glycidyl ether, nonyl glycidyl ether, undecyl glycidyl ether, tridecyl glycidyl ether, pentadecyl glycidyl ether, 2-ethylhexyl glycidyl ether, allyl glycidyl ether, phenyl glycidyl ether, cresyl glycidyl ether, t-butylphenyl glycidyl ether, dibromo phenyl glycidyl ether, benzyl glycidyl ether, polyethylene oxide glycidyl ether; glycidyl esters such as glycidyl formate, glycidyl acetate, glycidyl acrylate, glycidyl methacrylate, and glycidyl benzoate; and glycidyl amides.

Among the mono-functional epoxy compounds, because the water absorption ratio of regenerated collagen decreases, it is preferable to use a mono-functional epoxy compound represented by the following general formula (1). In the formula, R represents the same as defined above.

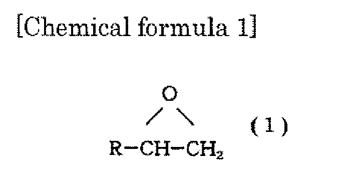

The regenerated collagen thus obtained is in the state of being swelled with water or the aqueous solution of inorganic salt. It is favorable that this swelled material contains water or the aqueous solution of inorganic salt in an amount of 4 to 15 times the weight of regenerated collagen. When the content of water or the aqueous solution of inorganic salt is 4 times or more, the content of aluminum salt in the regenerated collagen becomes high, and a sufficient water resistance is obtained. When the content is 15 times or less, the strength does not lower, and ease of handling is obtained.

The swelled regenerated collagen then is immersed in an aqueous solution of aluminum salt. The aluminum salt contained in the aqueous solution of aluminum salt preferably is a basic aluminum chloride or basic aluminum sulfate represented by the following formula: Al(OH)ₙCl₃₋ₙ, or Al₂(OH)₂ₙ(SO₄)₃₋ₙ, where n is 0.5 to 2.5. Specifically, examples that can be used include aluminum sulfate, aluminum chloride and alum. These aluminums can be used alone or in combination of two or more. The concentration of aluminum salt in the aqueous solution of aluminum salt preferably is 0.3 to 5 wt% on an aluminum oxide basis. When the concentration of aluminum salt is 0.3 wt% or more, the content of aluminum salt in the regenerated collagen fiber will be high and a sufficient water resistance will be obtained. When the content of aluminum salt is 5 wt% or less, the resultant will not be so hard even after treatment, and ease of handling is obtained.

The pH of the aqueous solution of aluminum salt is adjusted to usually, 2.5 to 5 using, for example, hydrochloric acid, sulfuric acid, acetic acid, sodium hydroxide, sodium carbonate or the like. When the pH is 2.5 or more, the structure of collagen can be maintained favorably. When the pH is 5 or less, precipitation of aluminum salt does not occur, and the solution will permeate uniformly. It is preferable that the pH first is adjusted to 2.2 to 3.5 so as to allow the aqueous solution of aluminum salt to permeate sufficiently into the regenerated collagen, and after that, for example, sodium hydroxide, sodium carbonate or the like is added to adjust the pH to 3.5 to 5, and the treatment is finished. When using a highly basic aluminum salt, only the first pH adjustment of 2.5 to 5 is performed. There is no particular limitation on the temperature of the aqueous solution of aluminum salt, but the solution temperature preferably is 50°C or lower. When the solution temperature is 50°C or lower, modification or alteration of the regenerated collagen does not occur easily.

The time during which the regenerated collagen is immersed in the aqueous solution of aluminum salt is 3 hours or more, and preferably 6 to 25 hours. With an immersion time of at least 3 hours, the reaction of the aluminum salt proceeds sufficiently, and regenerated collagen with a sufficient water resistance can be obtained. There is no particular limitation on the upper limitation for the immersion time, but as long as the immersion time is within 25 hours, the reaction of the aluminum salt proceeds sufficiently, and a favorable water resistance is obtained as well. In order to prevent nonuniform concentration, which is caused by the aluminum salt being absorbed quickly into the regenerated collagen, an inorganic salt, such as sodium chloride, sodium sulfate or potassium chloride, may be added to the aluminum salt as appropriate.

The cross-linked regenerated collagen treated with an aluminum salt as described above then is subjected to washing with water, oiling and drying. The regenerated collagen fiber thus obtained has little color, unlike the collagen fiber obtained by a conventional method of treating with a chromium salt, and also has a superior water resistance. Generally speaking, in order to prevent collagen from modification (gelatinization), care needs to be taken with the temperature history during processing. In order to prevent modification even after the collagen is cross-linked, it is necessary to control moisture and temperature during production, powdering process/product storage to keep them below a level at which regenerated collagen is modified. When most of the collagen is gelatinized, its characteristics change, and thus it is difficult to obtain desired collagen characteristics. It is advantageous to use regenerated collagen described above in terms of modification prevention.

When forming fibers from the collagen solution, it is easy to perform coloring with a known method by mixing the solution with a pigment or colorant or by adding a pigment or colorant immediately before spinning. As for the pigment or colorant to be used, depending on applications, a pigment or colorant that does not leach/separate in the spinning step or the powdering step can be selected, or the type or color phase can be selected according to the required quality of products that employ the present invention. Where appropriate, a filler, an aging inhibitor, a flame retardant, an antioxidant, etc. can be added. Instead of the collagen fiber production step described above, it is also possible to produce a film by using a slit nozzle in the same manner as described above, and make the film into powder.

In the present invention, the regenerated collagen obtained in the manner described above is pulverized, and thereby, a collagen powder made of cross-linked regenerated collagen (regenerated collagen powder) can be obtained. When the regenerated collagen is in the form of a fiber or film, it is cut into a fiber length or size suitable for pulverizing, or the cut fiber or film is pulverized further. Alternatively, the fiber or film is directly pulverized. Thus, a regenerated collagen powder can be obtained. There is no particular limitation on the cutter that can be used in the production of the regenerated collagen powder. For example, the fiber or film is cut into approximately 0.1 mm to several mm using a cutter that is used conventionally to cut fibers, such as a blade rotary cutter, belt cutter, shearing machine or cutter mill. The resultant is pulverized into fine particles using a pulverizing machine, for example, a shearing mill such as a roller mill, rod mill, ball mill (dry type, wet type), jet mill, pin mill, vibration mill, centrifugal (CF) mill, planetary ball mill or grinder mill, or pulverized into ultra-fine particles using a medium agitation type ultra-fine pulverizing machine or the like. From the viewpoint of preventing the ball material from mixing with powders and achieving pulverization efficiency, it is preferable to use hard balls such as zirconia balls. It is also possible to use balls of other materials such as alumina balls. As another pulverization method, freeze pulverization can be used as well.

It is preferable that the aluminum salt-containing resin powder thus obtained has an average particle size of 0.01 to 80 µm. The average particle size can be measured using a commercially available particle size distribution analyzer. The measurement can be performed using, for example, Microtrac particle size distribution analyzer (MT3300 available from Nikkiso Co., Ltd.) by laser diffraction/scattering method, or the like.

It is preferable that the content of aluminum in the aluminum salt-containing resin powder is within a range ranging from 0.1 to 70 wt% on a metallic simple substance basis, more preferably a range ranging from 0.2 to 50 wt%, and even more preferably a range ranging from 1 to 40 wt%.

The aluminum salt-containing resin powder of the present invention has a phosphorus adsorption capability. There is no particular limitation on phosphorus to be adsorbed as long as it contains elemental phosphorus or is a phosphorus compound. The aluminum salt-containing resin powder can adsorb, for example, a phosphoric acid structure. As used herein, "phosphoric acid structure" refers to a substance having a phosphoric acid backbone such as phosphoric acid, a phosphoric acid salt and a phosphoric acid ester. Ordinarily, elemental phosphorus often exists in the form of a phosphoric acid structure in nature. As a preferred method by which the phosphorus adsorbent of the present invention adsorbs phosphorus, a method of simply mixing an aqueous solution containing phosphorus with the regenerated collagen powder, serving as a phosphorus adsorbent, or a phosphorus adsorbent that is a mixture of the phosphorus adsorbent and a base body can be used. In order to achieve more efficient adsorption, it is desirable to disperse the phosphorus adsorbent or the phosphorus adsorbent in the solution as uniformly as possible.

The phosphorus adsorbent of the present invention may be a mixture obtained by combining the below-described regenerated collagen powder and a base body made of various materials.

As the base body, it is possible to use various categorized materials such as an inorganic material, an organic material, a metal material, and a composite material obtained by combining two or more of these materials.

Specifically, examples of the inorganic material include ceramics such as calcium carbonate, aluminum hydroxide, mica, and glass. Examples of the organic material include: proteins such as cotton, hemp, wool, kenaf and softwood pulp; natural polymers such as cellulose; plastics such as polyethylene, polyester, polypropylene, nylon and rayon; and petroleum-based synthetic resin materials such as synthetic fiber. Examples of the metal material include copper, lead, aluminum, other metals, superconducting alloys, and new inorganic materials such as amorphous alloy, shape memory alloy and fine steel.

The regenerated collagen powder alone can adsorb phosphorus. Other methods include a method of combining with a base body, a method of mixing with regenerated collagen powder by kneading, a method of forming a compound by a chemical reaction, a method of mixing with other resin, or a method in which the regenerated collagen powder is placed in a container having appropriate holes so that the powder gradually is released into a solution to be adsorbed. These methods may be used alone or in combination.

There is no particular limitation on the ratio of the phosphorus adsorbent to the phosphorus adsorbent of the present invention as long as a phosphorus adsorption capability is obtained, but the ratio preferably is 0.1 to 99 wt%.

By using the mixture of the present invention as a material that adsorbs phosphorus contained in lakes, reservoirs, rivers or household drainage, it is possible to produce products of various forms that have a superior phosphorus adsorption property.

As the phosphorus adsorption method of the present invention, for example, the phosphorus adsorbent or phosphorus adsorbent of the present invention is placed in a container having holes of appropriate size, and the container is submerged in drainage such as lakes, reservoirs, rivers or wastewater from households. Thereby, phosphorus can be adsorbed. When adsorption is performed in a small area, holes having a size that does not allow the powder to go out from the container are sufficient, but when adsorption is performed in a wide area, it is desirable to use a container having holes whose size is adjusted such that the powder gradually is released from the container. They may be used alone or in combination.

By mixing the phosphorus adsorbent of the present invention with various base bodies or by incorporating it in a container, the phosphorus adsorbent of the present invention can be used as a phosphorus adsorbent that adsorbs phosphorus contained in lakes, reservoirs, rivers and household drainage such as wastewater from households for the purpose of clarifying lakes, reservoirs, rivers and household drainage such as wastewater from households.

As for the particle size of the regenerated collagen powder used in the present invention, a particle size of approximately 0.1 to several mm exhibits an antibacterial property, but by pulverization into a fine powder having an average particle size of 0.01 to 80 µm, advantages can be obtained such as the antibacterial property being improved further, and the powder can be used as a spray agent by mixing it with an aqueous medium, an organic solvent-based medium or the like.

It is possible to adjust appropriately the particle size of the resulting regenerated collagen powder by changing the type of pulverizing machine and the pulverization time. For example, when a vibration mill is used, particles having an average particle size of approximately 5 to 80 µm are obtained from an hour to several tens of hours, but in order to obtain particles having an average particle size of 0.01 to 5 µm, the pulverized regenerated collagen powder is sized.

When the finely powdered regenerated collagen is mixed with an aqueous medium, a organic solvent-based medium or the like to obtain a spray agent, by spraying the spray agent to the seats, mats and plastic components in automobiles, the curtains, mats, couches, carpets and clothes in houses, etc., the antibacterial or antifungal property can be imparted to these materials. It goes without saying that the applications of the spray agent are not limited to the examples given above.

The regenerated collagen powder thus obtained has a superior antibacterial property or antifungal property against microorganisms that exist in the environment in which humans live, and therefore it can be used as an antibacterial agent or antifungal agent to be added to resins. Accordingly, by using the regenerated collagen powder, a resin composition having a superior antibacterial property or antifungal property can be obtained without blending in another antibacterial agent or antifungal agent.

The antibacterial property or antifungal property of the collagen powder made of cross-linked regenerated collagen used in the present invention is a characteristic property that cannot be found in regenerated collagen obtained through extraction, by a known method, from an animal raw material such as bovines, pigs, horses, deers, rabbits, birds and fish.

Furthermore, the regenerated collagen powder has superior dispersibility (resin dispersibility) in synthetic resins or solvents, and thus it is not always necessary to perform preliminary dispersion in the working process. This is presumably because the regenerated collagen has both hydrophilic and hydrophobic groups and is very strongly cross-linked, and consequently, the heat resistance and water resistance are high and it is not so much influenced by heat, moisture and solvents, and as a result, the viscosity on the surface is low, and the aggregation or association of powder particles does not occur easily. In the present invention, because the regenerated collagen powder is superior in terms of resin dispersibility, it is possible to obtain resin articles of various forms that have a superior antibacterial property or antifungal property.

Similarly to conventional collagen, the regenerated collagen powder also has a superior resin modification effect. The resin modification effect includes heat resistance, water resistance, formaldehyde adsorption property, moisture absorption/desorption property, wettability reducing effect, delustering effect, etc.

The antibacterial agent or antifungal agent of the present invention includes regenerated collagen powder described above, and has a superior antibacterial property or antifungal property, and resin dispersibility effected by the regenerated collagen powder, and also has a resin modification effect. The antibacterial agent or antifungal agent of the present invention may be mixed with other components as long as the antibacterial property or antifungal property, the resin dispersibility, and if necessary, the resin modification effect are not impaired. Examples of other components to be mixed include, but are not limited to, a film-forming agent, an ultraviolet shielding agent, an agent having an electromagnetic wave shielding effect, etc.

In the present invention, it is also possible to use carboxymethyl cellulose and polyvinyl alcohol as the matrix resin. Both carboxymethyl cellulose and polyvinyl alcohol are water-soluble matrix resin gel components before being cross-linked, and by bringing them into contact with an aluminum salt, they are cross-linked and the aluminum salt is bonded chemically to the resin gel component, and as a result, a water insoluble resin can be obtained. Specifically, carboxymethyl cellulose can be cross-linked with an aluminum salt because it has a -COOH group and a -OH group. Likewise, polyvinyl alcohol can be cross-linked with an aluminum salt because it has an -OH group. As the polyvinyl alcohol, a polyvinyl alcohol to which a -COOH group has been introduced may be used. The amount of -COOH group introduced can be, for example, approximately 0.1 to 5 mol%.

As the carboxymethyl cellulose, for example, "carboxymethyl cellulose sodium salt" available from SIGMA Corporation can be used. As the polyvinyl alcohol, for example, "anion-modified PVA (A series)" (grade: AF17) available from Japan VAM and POVAL Co., Ltd. can be used.

By mixing the antibacterial agent or antifungal agent thus obtained with a resin material, a resin composition having a superior antibacterial property or antifungal property can be obtained. Furthermore, the resin composition of the present invention is superior in terms of heat resistance, water resistance, formaldehyde adsorption property, moisture absorption/desorption property, wettability, delustering, etc. The amount of the antibacterial agent or antifungal agent added preferably is 0.1 wt% to 80 wt% relative to the total amount of the resin composition. The amount of the antibacterial agent or antifungal agent added can be adjusted within a range that the effects obtained by addition of the agent, such as a moisture absorption/desorption property and a formaldehyde adsorption property, are obtained, and that resin characteristics also are obtained and cost efficiency is satisfied.

Because the resin composition of the present invention employs the antibacterial agent or antifungal agent having the characteristics of both an antibacterial agent or antifungal agent and a resin modifying agent, it is unnecessary to add additionally another antibacterial agent or antifungal agent, such as an Ag-containing composition or compound or a pyridine-based compound. Thus, the cost can be reduced. A combined use of the resin composition of the present invention with another antibacterial agent or antifungal agent is of course possible.

As the resin material, it is preferable to use a composition containing at least one resin selected from the group consisting of polyamide resin, vinyl chloride resin, polyurethane resin, polyester resin, polyacrylic resin, styrene resin, acrylic silicone-based resin, epoxy ester resin, fluorine-based resin, polyolefin-based elastomer, polyester-based elastomer, and styrene-based elastomer. A combined use thereof is possible as long as the characteristics of the regenerated collagen powder, that is, a moisture absorption/desorption property, a chemical substance adsorption property and the like, are not impaired. It is also possible to add a filler, an aging inhibitor, a flame retardant, an antioxidant, etc. where appropriate.

Mixing of the antibacterial agent or antifungal agent, the resin material, and optionally other components can be performed using known methods for producing a resin composition. There is no particular limitation on the mixing conditions as long as known conditions are used.

By using the resin composition of the present invention as a coating material, artificial leather, synthetic leather, or molding material, it is possible to produce products of various forms that have a superior antibacterial property or antifungal property.

It is also possible to mix the antibacterial agent or antifungal agent with the resin material to obtain a resin composition, and the resin composition can be used to sterilize bacteria or mold.

There is no particular limitation on the products as long as they can be produced from resin compositions. Examples include products that come in frequent contact with humans, specifically, interior materials for vehicles, aircraft and ships such as handles and seats, outdoor materials for furniture such as couches and chairs, stretchable materials such as cushion materials, materials for daily commodities such as leather-like clothes, bags, pouches, shoes, leather-like gloves, tableware and stationery, decoration materials for interior decoration, components of electrical appliances such as cell phones and personal computers, and filters.

The product shape can be a sheet shape produced by injection extrusion, kneading, a film-forming method, or the like (hereinafter referred to as a "sheet shape").

There exist bacteria and fungi, but materials that are effective against both of them are scarce. Accordingly, there is a demand for materials having such a function. Generally speaking, bacteria are roughly classified into the following: Gram positive bacteria having a large amount of peptidoglycan on the cell walls; Gram negative bacteria having lipopolysaccharide; and other bacteria. Furthermore, Gram positive bacteria are classified into Gram positive cocci and Gram positive bacilli.

Gram positive cocci include facultative anaerobic cocci and aerobic cocci. The genera include the genus Micrococcus, the genus Staphylococcus, the genus Streptococcus, and the genus Enterococcus. As pathogens, Staphylococcus aureus and methicillin-resistant Staphylococcus aureus (MRSA) of the genus Staphylococcus, and Streptococcus pyogenes, Group B Streptococcus, Streptococcus pneumoniae, and Streptococcus viridans of the genus Streptococcus are known.

Gram positive bacilli are classified into the genus Corynebacterium, the genus Listeria, the genus Erysipelothrix, the genus Bacillus, and the genus Mycobacterium. As major pathogens, diphtheria bacillus of the genus Corynebacterium, Listeria monocytogenes of the genus Listeria, Erysipelothrix rhusiopathiae of the genus Erysipelothrix, Bacillus anthracis and Bacillus cereus of the genus Bacillus, and Mycobacterium tuberculosis of the genus Mycobacterium are known.

As Gram negative bacteria, Gram negative bacilli are a major group.

As Gram negative bacilli, there are aerobic Gram negative bacilli and Gram negative facultative anaerobic bacilli.

As major genera of aerobic Gram negative bacilli, there are Pseudomonas, Burkholderia, Rastonia, Legionella, Brucella, Bordetella, Alcaligenes, Francisella, etc. As pathogens, Pseudomonas aeruginosa of the genus Pseudomona, Legionella pneumophila of the genus Legionella, Brucella melitensis, Brucella abortus and Brucella suis of the genus Brucella, etc. are known.

Gram negative facultative anaerobic bacilli are classified into the family Enterobacteriaceae, the family Vibrionaceae, and the family Pasteurella. The family Enterobacteriaceae further is classified into the genus Escherichia coli, the genus Klebsiella, the genus Serratia, the genus Proteus, and the genus Yersinia. As pathogens, Escherichia coli, such as O157, of the genus Escherichia coli, Salmonella, Shigella, Klebsiella pneumoniae of the genus Klebsiella, Serratia marcecence of the genus Serratia, Proteus vulgaris and Proteus mirabilis of the genus Proteus, and Yersinia pestis of the genus Yersinia are known. Also, among the family Vibrionaceae, Vibrio cholerae of the genus Vibrio is known as a pathogen, and among the family Pasteurella, Pasturella multocida of the genus Pasteurella is known as a pathogen.

Other bacteria include obligatory anaerobic bacteria and the spirillum group, which are groups in which both Gram positive and negative bacteria are included. The following bacteria are known.

Obligatory anaerobic bacteria are classified into obligatory spore-forming bacteria, obligatory anaerobic Gram positive asporogenous bacilli, obligatory anaerobic Gram negative asporogenous bacilli, anaerobic Gram positive cocci, and anaerobic Gram negative cocci. As pathogens, among obligatory spore-forming bacteria, there are Clostridium tetani, Clostridium botulinum, Clostridium perfringens, Clostridium difficile, etc.

Among the spirillum group, C. fetus, C. jejuni and C. colit of the genus Campylobacter are known as pathogens.

The above-listed bacteria are known as pathogens that cause various diseases. Particularly, the following bacteria are often found in food poisoning cases and hospital infections: Escherichia coli, Staphylococcus aureus, Pseudomonas aeruginosa, MRSA, Bacillus cereus, and Klebsiella pneumoniae, and thus they are extremely important as a target for antibacterial agents.

Next, fungi are classified roughly into yeasts and molds.

Molds are classified into the genus Aspergillus, the genus Penicillium, the genus Cladosporium, the genus Alternaria, the genus Fusarium, the genus Aureobasidium, the genus Trichoderma, and the genus Chaetomium. Examples of molds that are considered as a target for antibacterial agents include those listed in JIS Z 2911, namely, Aspergillus niger, Aspergillus terreusm and Eurotium tonophilum (the first group); Penicillium citrinum and Penicillium funiculosum (the second group); Rhizopus oryzae (the third group); Cladosporium cladosporioides (or Kurokawa mold), Aureobasidium pullulans and Gliocladium virens (the fourth group); Chaetomium globosum, Fusarium moniliforme and Myrothecium verrucaria (the fifth group), etc.

Yeasts are classified into the genus Candida, the genus Rhodotorula and the genus Saccharomyces.

The antibacterial agent of the present invention has the effect of inhibiting the growth of the above-listed bacteria and fungi corresponding to yeasts.

The antifungal agent of the present invention has the effect of inhibiting the growth of the above-listed fungi corresponding to molds.

The present invention relates to an antibacterial agent or antifungal agent, but the case in which the present invention has antibacterial and antifungal capabilities is not excluded, and the present invention can have both.

### Examples

Hereinafter, the present invention will be described in detail with reference to examples, but it is to be understood that the present invention is not limited to these examples. In the following examples, percent "%" indicates weight percent "wt%".

### (Production Example 1)

Bovine split hide was used as a raw material. Thirty grams of aqueous solution of hydrogen peroxide diluted to 30 wt% was introduced to 1200 kg (collagen content: 180 kg) of hide piece solubilized with alkali, and then the hide was dissolved in an aqueous solution of lactic acid to produce a raw material solution having a pH adjusted to 3.5 and a solid content adjusted to 7.5 wt%. The raw material solution was agitated and deaerated by agitation/deaeration machine (8DMV Type, available from Dalton Co., Ltd.) under a reduced pressure, delivered to a piston type spinning solution tank, allowed to stand in a reduced pressure, and deaeration was performed. This raw material solution was extruded by the piston, delivered in a fixed amount by a gear pump, and filtered with a sintered filter having a pore size of 10 µm. After that, the solution was passed through a spinning nozzle having 300 pores with a pore size of 0.275 mm and a pore length of 0.5 mm, and discharged into a 25°C spinning bath (having a pH adjusted to 11 with boric acid and sodium hydroxide) containing 20 wt% of sodium sulfate at a spinning rate of 5 m/min.

Then, the obtained regenerated collagen fibers (300 fibers, 20 m) were immersed in 1.32 kg of aqueous solution containing 1.7 wt% of epichlorohydrin, 0.0246 wt% of sodium hydroxide and 17 wt% of sodium sulfate 17 for 4 hours at 25°C. After that, the temperature of the reaction solution was increased to 43°C to impregnate the fibers with the solution for 2 hours.

The reaction solution was removed after completion of the reaction, and then batch washing was performed three times using 1.32 kg of 25°C water in a flow type apparatus. After that, the fibers were impregnated with 1.32 kg of aqueous solution containing 5 wt% of aluminum sulfate, 0.9 wt% of trisodium citrate salt and 1.2 wt% of sodium hydroxide at 30°C, and 13.2 g of 5 wt% aqueous solution of sodium hydroxide was added to the reaction solution 2 hours, 3 hours and 4 hours after the start of the reaction, and the reaction was continued for 6 hours in total. The reaction solution was removed after completion of the reaction, and then batch washing was performed three times using 1.32 kg of 25°C water in a flow type apparatus.

Subsequently, part of the produced fibers was immersed in a bath filled with an oil solution made of an emulsion of amino modified silicone and a pluronic type polyether-based antistatic agent to cause the oil solution to adhere. In a hot-air convection dryer set at 50°C, one end of the fiber bundle was fixed, and a 2.8 g weight per fiber was attached to the other end and suspended. Drying was performed under tension for 2 hours, and regenerated collagen fibers of 60 deci tex were obtained.

The obtained regenerated collagen fibers were physically pulverized. Specifically, first, 2 kg of the regenerated collagen fibers was cut into a length of around 1 mm using a cutter mill SF-8 (available from Sanriki Seisakusho Co., Ltd.), and collected using a cyclone CYC-600 type (available from Sanriki Seisakusho Co., Ltd.). The cut pieces were used in an antibacterial activity test against Staphylococcus aureus performed in Example 1 described later. Next, pulverization was performed using a vibration mill (available from Token Co.). As conditions for pulverization, the cut collagen fibers, with a filling capacity of 40% (500 g), were put into a 4 liter alumina container containing alumina balls (diameter: 19 mm) with a filling capacity of 80%, and pulverization was carried out for 4 to 12 hours. As a result, powders having an average particle size of 33 µm were obtained through pulverization for 4 hours, and powders having an average particle size of 13 µm were obtained through pulverization for 12 hours. These powders were used for an antibacterial activity test against Escherichia coli performed in Example 1 described later.

### (Production Example 2)

A resin composition of Production Example 2 contains an olefin-based thermoplastic elastomer, the regenerated collagen powder and silicone, and has the following composition: 90 parts by weight of an olefin-based thermoplastic elastomer (TPO available from Sumitomo Chemical Co., Ltd. (trade name: Sumitomo TPE3675)), 10 parts by weight of the regenerated collagen powder (average particle size: 13 µm and 33 µm), and 2 parts by weight of silicone (silicone rubber of Dow Corning Silicone Toray Co., Ltd., trade name: SE6749U).

The resin composition was melted and kneaded three times (10 minutes) at 140°C on two rotating rollers of an apparatus (Nippon Roll MFG. Co., Ltd.) to obtain a 250 µm thick sheet. In addition, a sheet-shaped product having a thickness of about 260 µm was obtained in the same manner, except that, in the above composition, the amount of the olefin-based thermoplastic elastomer (TPO available from Sumitomo Chemical Co., Ltd. (trade name: Sumitomo TPE3675)) was changed to 80 parts by weight and the amount of the regenerated collagen powder was changed to 20 parts by weight.

### (Production Example 3)

A resin composition of Production Example 3 contains an emulsion type aqueous acrylic silicone resin, the regenerated collagen powder and an additive, and has the following composition.

One hundred parts by weight of an emulsion type aqueous acrylic silicone resin (available from Kaneka Corporation, solid content: 50% (trade name: Gemlac W#3108F)), 10 parts by weight of the regenerated cross-linked collagen, and 3 parts by weight of a film-forming agent texanol (available from Chisso Corporation, trade name: CS12).

The resin composition was agitated and mixed in the presence of glass beads having a diameter 1 mm for about 10 minutes, applied to a glass plate (length: 150 mm, width: 70 mm, thickness: 0.75 mm) with an applicator or bar coater, and dried at room temperature for 5 hours to obtain a coated sample. In addition, a coated sample was obtained with the same operation except that, in the above composition, aqueous polyurethane resin AQD-473WX02 (available from Nippon Polyurethane Industry Co., Ltd.) was used in the same amount instead of the emulsion type acrylic silicone resin.

### (Production Example 4)

A resin composition of Production Example 4 contains a one component type polyurethane solvent-based coating material, the regenerated collagen powder and additives, and has the following composition.

One hundred parts by weight of a one component type polyurethane resin Nippollan 5199 (available from Nippon Polyurethane Industry Co., Ltd., solid content: 30%), 10 parts by weight of the regenerated collagen powder, 30 parts by weight of a solvent such as toluene or DMF, and 3 parts by weight of a film-forming agent texanol (available from Chisso Corporation, trade name: CS12).

The resin composition was agitated and mixed in the presence of glass beads having a diameter 1 mm for about 10 minutes, applied to a glass plate with an applicator or bar coater, and dried to obtain a coated sample. In addition, a coated sample was obtained by the same operation except that, in the above composition, a one component type polyurethane resin Resamine ME-3612LP (available from Dainichiseika Color & Chemicals Mfg. Co., Ltd.) was used in the same amount instead of the one component type polyurethane resin Nippollan 5199.

### (Production Examples 5 to 7)

Samples of Production Examples 5 to 7 (Production Example 5 corresponding to Production Example 2 without the regenerated collagen powder, Production Example 6 corresponding to Production Example 3 without the regenerated collagen powder, and Production Example 7 corresponding to Production Example 4 without the regenerated collagen powder) were produced by the same procedure as Production Examples 2 to 4, respectively, except that the regenerated collagen powder was not added.

### Production Example 5

A resin composition of Production Example 5 contains an olefin-based thermoplastic elastomer and silicone, and has the following composition.

One hundred parts by weight of an olefin-based thermoplastic elastomer (TPO available from Sumitomo Chemical Co., Ltd.: Sumitomo TPE3675) and 2 parts by weight of silicone (silicone rubber of Dow Corning Silicone Toray Co., Ltd., trade name: SE6749U).

The resin composition was melted and kneaded three times (about 10 minutes) at 140°C on two rotating rollers of an apparatus (Nippon Roll MFG. Co., Ltd.) to obtain a 250 µm thick sheet.

### Production Example 6

A resin composition of Production Example 6 is a resin composition in which a desired additive is added to an emulsion type aqueous acrylic silicone resin, and has the following composition.

One hundred parts by weight of an emulsion type aqueous acrylic silicone resin (available from Kaneka Corporation, solid content: 50% (trade name Gemlac W#3108F)), and 3 parts by weight of a film-forming agent texanol (available from Chisso Corporation, trade name: CS12)

The resin composition was agitated and mixed in the presence of glass beads for about 10 minutes, applied to a glass plate (length: 150 mm, width: 70 mm, thickness: 0.75 mm) with an applicator or bar coater, and dried to obtain a coated sample. In addition, a coated sample was obtained with the same operation except that, in the above composition, aqueous polyurethane resin AQD-473EX02 (available from Nippon Polyurethane Industry Co., Ltd.) was used in the same amount instead of the emulsion type acrylic silicone resin.

### Production Example 7

A resin composition of Production Example 7 is a resin composition in which desired additives are added to a one component type polyurethane solvent-based coating material, and has the following composition.

One hundred parts by weight of a one component type polyurethane resin Nippollan 5199 (available from Nippon Polyurethane Industry Co., Ltd., solid content: 30%), 30 parts by weight of a solvent such as toluene or DMF, and 1 part by weight of a dispersing agent.

The resin composition was agitated and mixed in the presence of glass beads having a diameter 1 mm for about 10 minutes, applied to a glass plate with an applicator or bar coater, and dried to obtain a coated sample. In addition, a coated sample was obtained by the same operation except that, in the above composition, a one component type polyurethane resin Resamine ME-3612LP (available from Dainichiseika Color & Chemicals Mfg. Co., Ltd.) was used in the same amount instead of the one component type polyurethane resin Nippollan 5199.

### (Production Example 8)

A resin composition of Production Example 8 is a resin composition in which a one component type polyurethane solvent-based coating material, the regenerated collagen powder and additives are added, and has the following composition.

One hundred parts by weight of a one component type polyurethane resin Nippollan 5199 (available from Nippon Polyurethane Industry Co., Ltd.), 10 parts by weight of the regenerated collagen powder, 30 parts by weight of a solvent such as toluene or DMF, and 3 parts by weight of a film-forming agent texanol (available from Chisso Corporation, trade name: CS12).

The resin composition was agitated and mixed in the presence of glass beads having a diameter 1 mm for about 10 minutes, applied to a TPO sheet with an applicator or bar coater, and dried to obtain a coated sample. In addition, a coated sample was obtained with the same operation except that, in the above composition, a one component type polyurethane resin Resamine ME-3612LP (available from Dainichiseika Color & Chemicals Mfg. Co., Ltd.) was used in the same amount instead of the one component type polyurethane resin Nippollan 5199.

### (Production Example 9)

A resin composition of Production Example 9 is a resin composition in which a one component type polyurethane solvent-based coating material, a silk powder and additives are added, and has the following composition..

One hundred parts by weight of a one component type polyurethane resin Nippollan 5199 (available from Nippon Polyurethane Industry Co., Ltd.), 10 parts by weight of a silk powder, 30 parts by weight of a solvent such as toluene or DMF, and 3 parts by weight of a film-forming agent texanol (available from Chisso Corporation, trade name: CS12).

The resin composition was agitated and mixed in the presence of glass beads having a diameter 1 mm for about 10 minutes, applied to a TPO sheet with an applicator or bar coater, and dried to obtain a coated sample. In addition, a coated sample was obtained with the same operation except that, in the above composition, a one component type polyurethane resin Resamine ME-3612LP (available from Dainichiseika Color & Chemicals Mfg. Co., Ltd.) was used in the same amount instead of the one component type polyurethane resin Nippollan 5199.

### (Production Example 10)

An insolubilized CMC powder was produced in the following manner. A 1% aqueous solution of carboxymethyl cellulose sodium salt (CMC; available from SIGMA Corporation) was produced, and the solution was dripped to an aluminum sulfate solution for aluminum cross-linking to form an insoluble material. The insoluble material was collected and dried, and after that, pulverized using a mortar to obtain fine particles.

### (Production Example 11)

An insolubilized PVA powder was produced in the following manner. A 10% (W/V) aqueous solution of anion-modified polyvinyl alcohol (available from Japan VAM and POVAL Co., Ltd., trade name: AF-17) was produced, and the solution was dripped to an aluminum sulfate solution for aluminum cross-linking to form an insoluble material. The insoluble material was collected and dried, and after that, pulverized using a mortar to obtain fine particles.

### (Quantitation of Aluminum)

Quantitation of aluminum as a metallic simple substance was performed, in the case of fibers, using regenerated collagen that had been cross-linked with an aluminum salt and then subjected to washing with water, oiling and drying, and in the case of powders, using regenerated collagen that had been pulverized. The regenerated collagen was subjected to wet oxidation degradation, and after that, measurement was performed by atomic absorption spectrometry. The measurement for regenerated collagen of other forms such as a film was performed in the same manner. As used herein, "aluminum as a metallic simple substance" refers only to aluminum atoms and an association thereof.

### (Example 1)

The antibacterial property of the powders of the cut regenerated collagen obtained in Production Example 1 against Staphylococcus aureus (NBRC12732 strain) was examined in the following manner.

Specifically, based on the quantitative test method for fiber products in accordance with JIS L-1902, approximately 3.4 × 10⁴ Staphylococcus aureus were inoculated into 0.2 mL of a culture medium to which 0.4 g of the regenerated collagen powder had been added, and incubated at 36°C for 18 hours. Then, the number of bacteria was counted. For comparison, incubation was performed in the same manner, except that the regenerated collagen powder was not added.

In the comparison in which the regenerated collagen powder was not added, the number of bacteria increased to 1.1 × 10⁷, whereas when the regenerated collagen powder was added, the number of bacteria decreased significantly to 3.3 × 10². As used herein, the number of bacteria refers to the number of bacteria present in the culture medium, and was calculated by counting the number of bacteria present in a predetermined amount of the culture medium with a microscope (the same applies hereinafter).

Also, the antibacterial property against Escherichia coli (E. coli. JM109 strain) of the regenerated collagen powder obtained in Production Example 1 was examined in the following manner.

Specifically, based on the quantitative test method for fiber products in accordance with JIS L-1902, approximately 1 × 10⁶ was inoculated into 10 mL of a culture medium to which 0.1 g of the regenerated collagen powder had been added, and incubated at 37°C for 6 hours. Then, the number of bacteria was counted. For comparison, incubation was performed in the same manner, except that the regenerated collagen powder was not added.

In the comparison in which regenerated collagen powder was not added, the number of Escherichia coli increased to 3.1 × 10⁸, whereas when the regenerated collagen powder was added, the number of bacteria decreased significantly to 1.4 × 10⁴.

The foregoing illustrates that the regenerated collagen powder of the present invention has a sterilizing property against pathogens transmitted through contact with Staphylococcus aureus, Escherichia coli, etc.

### (Examples 2 to 3 and Comparative Example 1)

A moisture absorption/desorption property test and a formaldehyde adsorption property test were performed for, as Example 2, two sheet-shaped samples (Example 2: average particle size of 33 µm, and Example 3: average particle size of 13 µm) produced in Production Example 2 by adding the regenerated collagen powder to an olefin-based thermoplastic elastomer, and as Comparative Example 1, the sheet-shaped sample produced in Production Example 5.

The moisture absorption/desorption property test was performed by measuring the amounts of increase and decrease in the weight of the sheet-shaped sample over time when the humidity was changed between 52% and 79% (JIS6544). Increases and decreases in the sample weight corresponding to the change in humidity were observed for the two sheet-shaped samples in which the regenerated collagen powder had been added. The results are shown in Table 1.

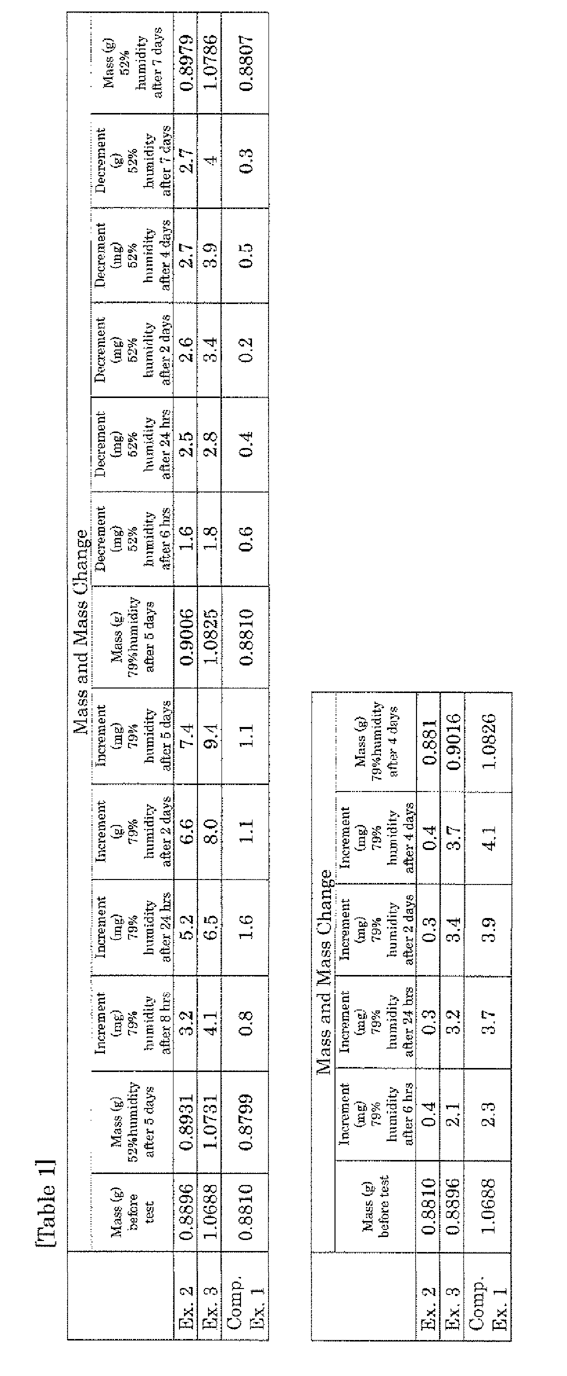

The formaldehyde adsorption property test was performed by the gasbag method. Specifically, the sample was introduced into a 3 L Tedlar bag. After degassing, 1.5 L of air whose formaldehyde concentration had been adjusted to 24 ppm in advance was added, and the contents were agitated by slightly shaking the Tedlar bag. After that, the concentration of formaldehyde was measured using a detector tube (Gastec Corporation 91L) after a predetermined period of time. As a result, both two samples that were produced by adding the regenerated collagen powder exhibited a significantly stronger formaldehyde adsorption property than the sample produced without addition of the regenerated collagen powder (Table 2). In other words, it was found that adding the regenerated collagen powder provides a favorable moisture absorption/desorption property and a favorable formaldehyde adsorption property.

**[Table 2]**

| Sample | Formaldehyde Adsorption Test Concentration of Formaldehyde in Tetrabag, ppm (on formaldehyde mass basis) | | | |
|---|---|---|---|---|
| | Initial Concentration | After 2 hrs | After 24 hrs | After 72 hrs |
| Ex. 2 | 18.0 | 14.4 | 5.0 | 2.3 |
| Ex. 3 | 18.0 | 13.0 | 4.0 | 1.8 |
| Comp. Ex. 1 | 18.0 | 16.0 | 8.0 | 4.0 |
| Ref. Ex. | 18.0 | 14.4 | 10.1 | 6.0 |

| | | | | |
|---|---|---|---|---|
| Note: only a container for Reference Example. | | | | |

### (Example 4 and Comparative Example 2)

A moisture absorption/desorption property test and a formaldehyde adsorption property test were performed for, as Example 4, the coated sample produced in Production Example 3 by adding the regenerated collagen powder (average particle size: 13 µm) to an emulsion type aqueous acrylic silicone resin, and as Comparative Example 2, the coated sample produced in Production Example 6.

The moisture absorption/desorption property test and the formaldehyde adsorption property test were performed by the same procedure as in Example 2. An increase was observed in the sample weight (Table 3), and an increase also was observed in the formaldehyde adsorption (Table 4). In other words, it was found that adding the regenerated collagen powder provides a favorable formaldehyde adsorption property and a favorable moisture absorption/desorption property, and a texture and characteristics similar to those of leather are obtained.

**[Table 3]**

| | Adsorption test Increased mass when humidity is changed from 52% to 79% | Note |
|---|---|---|
| Ex. 4 | 5.0 mg | Number of sheets tested: 1 |
| Comp. Ex. 2 | 3.2 mg | Number of sheets tested: 1 |

| | | |
|---|---|---|
| Note: the test was performed in accordance with JTS K 6544. Hide powder test was performed by placing hide powders in an aluminum container (upper opening diameter: φ 60 mm, bottom diameter: φ 50 mm, height: 30 mm) such that the powders were laid as flat as possible. The average of four points was used as a test value. | | |

**[Table 4]**

| Sample Initial | Formaldehyde Adsorption Test: Concentration of Formaldehyde in Tetrabag, ppm (mass on formaldehyde basis in Tedlar bag, mg) | | | | Note |
|---|---|---|---|---|---|
| | Concentration | After 2 hrs | After 24 hrs | After 72 hrs | |
| Ex. 4 | 24 ppm (0.048 mg) | 13.7 ppm (0.028 mg) | 3.3 ppm (0.007 mg) | 2.4 ppm (0.005 mg) | Number Tested: 2 |
| Comp. Ex. 2 | 24 ppm (0.048 mg) | 15.3 ppm (0.031 mg) | 8 ppm (0.016 mg) | 6 ppm (0.012 mg) | Number Tested: 2 |

Note: the test was performed by gasbag method. A sample was introduced into a 3 liter Tedlar bag. After degassing, 1.5 liter of air whose formaldehyde concentration had been adjusted to 24 ppm in advance was added, and the contents were agitated by slightly shaking the Tedlar bag. After that, the concentration of formaldehyde was measured using a detector tube (91L available from Gastec Corporation) after a predetermined period of time. The average of two points was used as a test value.

### (Examples 5 to 7 and Comparative Examples 3 to 5)

A wettability test and a luster test were performed for, as Example 5, the sheet-shaped sample produced in Production Example 2 by adding the regenerated collagen powder (average particle size: 13 µm) to an olefin-based thermoplastic elastomer, as Example 6, the coated sample of Production Example 3 produced by adding the regenerated collagen powder to an emulsion type aqueous acrylic silicone resin, as Example 7, the polyurethane resin sample of Production Example 4, and as Comparative Examples 3 to 5, the samples produced in Production Examples 5 to 7.

As a result, all of the samples of the examples had a larger contact angle than the comparative examples, and a decrease was observed at a reflection angle of 60 degrees (Table 5). In other words, it was found that adding the regenerated collagen powder provides a wettability reducing effect and a delustering effect.

**[Table 5]**

| Sample shape | Contact Angle (°) | | Luster Ratio (%: Reflection angle of 60°) | |
|---|---|---|---|---|
| Sheet | Ex. 5 | Comp. Ex. 3 | Ex. 5 | Comp. Ex. 3 |
| (Olefin-based thermoplastic elastomer) | 121 | 115 | 2.8 | 4.4 |
| Coating | Ex. 6 | Comp. Ex. 4 | Ex. 6 | Comp. Ex. 4 |
| (Emulsion type acrylic silicone resin-based resin) | 95 | 80 | 13.7 | >100 |
| Coating | Ex. 7 | Comp. Ex. 5 | Ex. 7 | Comp. Ex. 5 |
| (Polyurethane solvent-based resin) | 80 | 34 | 13.1 | >100 |

### (Example 8, Examples 9 to 12, Comparative Example 6, Comparative Examples 7 to 10)

For the regenerated collagen powder obtained in Production Example 1, as an antibacterial property test, antibacterial properties against E. coli JM109 strain, Bacillus cereus IFO13494, Pseudomonas aeruginosa NBRC13275, Staphylococcus aureus subsp- aureus NBRC12732, and Cladosporium cladosporioides IFO6348 (or Kurokawa mold) were evaluated, and the minimal inhibitory concentrations were calculated.

### (Growth Evaluation Method in Liquid Culture Medium)

The turbidity of a culture medium was checked visually, and when the culture medium turned turbid, it was defined as "Growth observed". When the culture medium was transparent, it was defined as "No growth".

### (Growth Evaluation Method in Flat Plate Culture Medium)

The area of a flat plate to which bacteria or fungi had been applied was checked visually. In comparison with a reference flat plate culture medium, when the growth of the bacteria was recognized visually in the entire applied area, it was defined as "Growth observed". When the growth of the bacteria was recognized visually in part of the applied area, it was defined as "Slight growth". When no difference was observed visually in the applied area, with reference to the reference flat plate, it was defined as "No growth".

### Example 8 (Escherichia coli), Comparative Example 6 (Escherichia coli)

In the test method of Example 8, the regenerated collagen powder obtained in Production Example 1 was used. Escherichia coli was inoculated into liquid culture mediums (L-broth, available from Difco Inc., yeast extract: 0.5%, bactopeptone: 1%, Nacl: 0.5%) to which the powder had been added in an amount of 2.5, 5.0, 7.5 and 10 (mg/ml), respectively, and incubated with shaking at 37°C overnight. After that, the presence/absence of growth was determined visually.

In Comparative Example 6, the test was performed in the same manner as in Example 8, except that the powder was not added to liquid culture mediums.

The results of Example 8 and Comparative Example 6 are shown in Table 6.

**[Table 6]**

| | Comp. Ex. 6 | Ex. 8 | | | |
|---|---|---|---|---|---|
| Added concentration of regenerated collagen powder (mg/ml) | 0 | 2.5 | 5 | 7.5 | 10 |
| Aluminum content (wt%) | 0 | 0.1 | 0.2 | 0.3 | 0.4 |
| Growth evaluation | Growth observed | Growth observed | No growth | No growth | No growth |

Example 9 (Bacillus cereus), Example 10 (Pseudomonas aeruginosa), Example 11 (Staphylococcus aureus), Comparative Example 7 (Bacillus cereus), Comparative Example 8 (Pseudomonas aeruginosa), Comparative Example 9 (Staphylococcus aureus)
For Bacillus cereus, Pseudomonas aeruginosa and Staphylococcus aureus strains, the following method was performed.

Specifically, as Examples 9 to 11, the powder was added to agar-containing culture mediums (Muller Hilton Agar available from Difco Inc.) maintained at 50°C such that the final concentration would be 3.125, 6.25, 12.5, 25, 50 and 100 (mg/ml), and sufficiently mixed. After that, the obtained mediums were injected into petri dishes, followed by solidification to produce flat plates for measurement. Next, each test strain was incubated in growing culture mediums (Pseudomonas aeruginosa: 0.4% potassium nitrate-added Muller Hilton Broth, and bacteria other than Pseudomonas aeruginosa Muller Hilton Broth) at 35°C for 20 hours. After that, the number (the number of bacteria in the case of bacteria) was adjusted to 10⁶/ml, and then they were spread onto the flat plates, and incubated at 35°C for 20 hours, and after that, for 7 days. Evaluation was performed based on minimal inhibitory concentrations, which are the minimum concentrations at which the growth of bacteria is inhibited.

As Comparative Examples 7 to 9, evaluation was performed in the same manner as in Examples 9 to 11, except that the powder was added to agar-containing culture mediums (Muller Hilton Agar available from Difco Inc.) maintained at 50°C such that the final concentration would be 0 (mg/ml).

The results of Examples 9 to 11 and Comparative Examples 7 to 9 are shown in Tables 7, 8 and 9. The regenerated collagen powder used here contained 40 wt% of aluminum on a metallic simple substance basis. "Added concentration of regenerated collagen powder: 100 mg/ml" shown in Tables 7 to 9 is equal to 4 wt% when expressed in an aluminum content on a metallic simple substance basis.

**[Table 7]**

| | Comp. Ex. 7 | Ex. 9 | | | | | |
|---|---|---|---|---|---|---|---|
| Added concentration of regenerated collagen powder (mg/ml) | 0 | 3.125 | 6.25 | 12.5 | 25 | 50 | 100 |
| Growth evaluation | Growth observed | Growth observed | Growth observed | Growth observed | Growth observed | No growth | No growth |

**[Table 8]**

| | Comp. Ex. 8 | Ex. 10 | | | | | |
|---|---|---|---|---|---|---|---|
| Added concentration of regenerated collagen powder (mg/ml) | 0 | 3.125 | 6.25 | 12.5 | 25 | 50 | 100 |
| Growth evaluation | Growth observed | Growth observed | Growth observed | No growth | No growth | No growth | No growth |

**[Table 9]**

| | Comp. Ex. 9 | Ex. 11 | | | | | |
|---|---|---|---|---|---|---|---|
| Added concentration of regenerated collagen powder (mg/ml) | 0 | 3.125 | 6.25 | 12.5 | 25 | 50 | 100 |
| Growth evaluation | Growth observed | Growth observed | Growth observed | No growth | No growth | No growth | No growth |

### (Example 12 (Kurokawa mold), Comparative Example 10 (Kurokawa mold))

For Kurokawa mold strain, the following method was performed.

Specifically, the powder was added to agar-containing culture mediums (sabouraud agar culture mediums available from Eiken Chemical Co., Ltd.) maintained at 50°C such that the final concentration would be 3.125, 6.25, 12.5, 25, 50 and 100 (mg/ml), and sufficiently mixed. After that, the obtained mediums were injected into petri dishes, followed by solidification to produce flat plates for measurement. Next, each test strain was incubated in a growing culture medium (Kurokawa mold: Potato Dextrose Agar available from Difco Inc.) at 25°C for 10 days. After that, the number (the number of spores in the case of Kurokawa mold) was adjusted to 10⁶/ml, and then they were spread onto the flat plates, and incubated at 25°C for 7 days, and after that, incubated. Evaluation was performed based on minimal inhibitory concentrations, which are the minimum concentrations at which the growth of bacteria is inhibited.

As Comparative Example 10, evaluation was performed in the same manner as in Examples 9 to 11, except that the powder was added to agar-containing culture mediums (sabouraud agar culture mediums available from Eiken Chemical Co., Ltd.) maintained at 50°C such that the final concentration would be 0 (mg/ml).

The results of Example 12 and Comparative Example 10 are shown in Table 10. The regenerated collagen powder used here contained 40 wt% of aluminum on a metallic simple substance basis. "Added concentration of regenerated collagen powder: 100 mg/ml" shown in Table 10 is equal to 4 wt% when expressed in an aluminum content on a metallic simple substance basis.

**[Table 10]**

| | Comp. Ex. 10 | Ex. 12 | | | | | |
|---|---|---|---|---|---|---|---|
| Added concentration of regenerated collagen powder (mg/ml) | 0 | 3.125 | 6.25 | 12.5 | 25 | 50 | 100 |
| Growth evaluation | Growth observed | Growth observed | Slight growth | No growth | No growth | No growth | No growth |

### (Examples 13, 14, 15 and Comparative Examples 11, 12)

For the emulsion type aqueous acrylic silicone resin-coated glass plate sample produced in Production Example 3 (by adding 10 parts by weight of the regenerated collagen powder having an average particle size of 33 microns), the solvent-based urethane resin-coated glass plate sample produced in Production Example 4 (by adding 10 parts by weight of the regenerated collagen powder having an average particle size of 33 microns), the solvent-based urethane resin-coated TPO sheet sample produced in Production Example 8 (by adding 10 parts by weight of the regenerated collagen powder having an average particle size of 13 microns), the solvent-based urethane resin-coated TPO sheet sample produced in Production Example 9 (by using a silk powder having an average particle size of 5 microns), and the glass sample coated with only a solvent-based urethane resin produced for comparison in Production Example 7, the presence/absence of antibacterial activity and the level of the activity were determined, and comparison was made.

Example 13: the emulsion type aqueous acrylic silicone resin-coated glass plate sample obtained by adding 10 parts by weight of the regenerated collagen powder having an average particle size of 33 microns.
Example 14: the solvent-based urethane resin-coated glass plate sample obtained by adding 10 parts by weight of the regenerated collagen powder having an average particle size of 33 microns.
Example 15: the solvent-based urethane resin-coated TPO sheet sample by adding 10 parts by weight of the regenerated collagen powder having an average particle size of 13 microns.
Comparative Example 11: the solvent-based urethane resin-coated TPO sheet sample obtained by using a silk powder having an average particle size of 5 microns.
Comparative Example 12: the glass sample coated with only a solvent-based urethane resin. As the antibacterial activity test, Escherichia coli (E.coli IFO 3972) was used as a test strain, and the test was performed in the following manner. Specifically, in accordance with JIS Z 2801, Escherichia coli was incubated with shaking in 5 ml of an ordinary broth culture medium (Eiken Chemical Co., Ltd.) at 27°C overnight. After that, the medium was diluted using a sterilized physiological salt solution containing an ordinary broth culture medium with a final concentration of 1/500. This bacteria solution in an amount of 0.4 ml was placed on a sheet sample housed in a container. The container was covered with a polyethylene sheet, and then allowed to stand at 30°C. The bacteria solution on the sample was collected at the time of inoculation and 24 hours after inoculation, and the number of viable bacteria was measured. The measurement of the number of viable bacteria was performed by diluting the bacteria solution stepwise, spreading it onto a flat plate culture medium and counting the number of colonies that appeared, in accordance with 1.2.1.1 general test method for bacteria written on page 59 of Methods of Analysis in Health Science 2005.

The results of Examples 13 to 15 and Comparative Examples 11 and 12 are shown in Table 11.

**[Table 11]**

| | Tested Bacterium | Sample | Measured | Number of viable bacteria (number/ml) |
|---|---|---|---|---|
| Ex. 13 | Escherichia coli | Emulsion type aqueous acrylic silicone resin/regenerated collagen powder (33 µ) | 24 hrs after inoculation | 1 × 10² |
| Ex. 14 | | Solvent-based polyurethane resin/regenerated collagen powder (33 µ) | 24 hrs after inoculation | 3.2 × 10² |
| Ex. 15 | | Solvent-based polyurethane resin/regenerated collagen powder (13 µ) | 24 hrs after inoculation | Not detected |
| Comp. Ex. 11 | | Solvent-based polyurethane resin/silk powder (5 µ) | 24 hrs after inoculation | 1.4 × 10⁷ |
| Comp. Ex. 12 | | Only solvent-based polyurethane | 24 hrs after inoculation | 5.6 × 10⁶ |
| | | | At inoculation | 1.1 × 10⁶ |

### (Example 16 and Comparative Example 8)

For the solvent-based urethane resin-coated TPO sheet sample produced in Production Example 8 and the solvent-based urethane resin-coated TPO sheet sample produced in Production Example 9 by using a silk powder, the presence/absence of antifungal activity was examined, and comparison was made.

The antifungal test was performed using Kurokawa mold (Cladosporium cladosporioides NBRC6348) in accordance with JIS Z 2911 (test method for mold resistance) 7.c (wet test method for textile products). Note that mold spores were suspended in a sabouraud culture medium to obtain a spore suspension). Specifically, the spore suspension was sprayed onto a sheet sample, and incubated at 25°C for 2 to 3 weeks. Evaluation was performed by observing the sheet surface with a stereo microscope.

The results of Example 16 and Comparative Example 13 are shown in Table 12.

**[Table 12]**

| | Tested Bacterium | Sample | Measured | Growth Evaluation |
|---|---|---|---|---|
| Ex. 16 | Kurokawa mold | Solvent-based polyurethane resin/ regenerated collagen powder (13 µ) | 3 weeks after inoculation | No growth |
| Comp. Ex. 13 | | Solvent-based polyurethane resin/ silk powder (5 µ) | 3 weeks after inoculation | Growth observed |

### (Examples 17 to 20)

The phosphorus adsorption capability of the regenerated collagen powder was examined in the following manner. The regenerated collagen powder was added to culture mediums (L-broth; Difco Inc., 0.5% yeast extract, Difco Inc. 1% bactopeptone, 0.5% NaCl) with a concentration range ranging from 0.25% (W/V) (Example 17), 0.5% (W/V) (Example 18), 1% (W/V) (Example 19), and 2% (W/V) (Example 20), and incubated while shaking at 37°C for 20 hours. After incubation, the medium was centrifuged at 1500 rpm for 5 minutes to collect the supernatant. Then, the amount of phosphorus contained in the collected liquid was measured. The amount of aluminum contained in the liquid also was measured on a metallic simple substance basis. The results are shown in Table 13. As shown in Table 13, the phosphorus content decreased in proportion to the amount of regenerated collagen powder added.

It should be noted that it was known in advance, from the composition analysis data of L-broth (obtained from Difco Inc.), that the phosphorus contained in the culture medium L-broth was mostly phosphorus derived from a phosphoric acid structure.

### (Comparative Example 14)

The phosphorus adsorption capability of the regenerated collagen powder was examined in the following manner. The regenerated collagen powder was added to culture mediums (L-broth; Difco Inc., 0.5% yeast extract, Difco Inc. 1% bactopeptone, 0.5% NaCl) with a concentration range of 0% (W/V), and incubated while shaking at 37°C for 20 hours. After incubation, the medium was centrifuged at 1500 rpm for 5 minutes to collect the supernatant. Then, the amount of phosphorus contained in the collected liquid was measured. The amount of aluminum contained in the liquid also was measured on a metallic simple substance basis. As a result, as shown in the table, the phosphorus content decreased in proportion to the amount of regenerated collagen powder added. The results are shown in Table 13.

It should be noted that it was known in advance, from the composition analysis data of L-broth (obtained from Difco Inc.), that the phosphorus contained in the culture medium L-broth was mostly phosphorus derived from a phosphoric acid structure.

**[Table 13]**

| | Added amount of regenerated collagen powder %(W/V) | Element | Measured value (ppm) |
|---|---|---|---|
| Example 17 | 0.25 | P | 32 |
| | | Al | 8.1 |
| Example 18 | 0.50 | P | 26 |
| | | Al | 14 |
| Example 19 | 1.00 | P | 21 |
| | | Al | 25 |
| Example 20 | 2.00 | P | 15 |
| | | Al | 37 |
| Comparative Example 14 | None | P Al | 710 |
| | | Al | 0 |

### (Example 21)

Allophane (Shinagawa Chemicals Co., Ltd.) known as a phosphorus adsorbent was obtained, and comparison was made between allophane and the regenerated collagen powder in terms of phosphorus adsorption capability. The method was basically the same as that of Example 19, except that the amounts of samples added (regenerated collagen powder and allophane) were changed to 1 wt% (W/V), and that centrifugation after incubation was performed at 3000 rpm for 10 minutes. The results are shown in Table 14. It was found that the regenerated collagen powder has a phosphorus adsorption capability superior to that of allophane.

### (Comparative Example 15)

Basically, the same method as that of Example 3 was performed, except that the amounts of samples added (regenerated collagen powder and allophane) were changed to 1 wt% (W/V), and that centrifugation after incubation was performed at 3000 rpm for 10 minutes. The results are shown in Table 14. It was found that the regenerated collagen powder has a phosphorus adsorption capability superior to that of allophane.

**[Table 14]**

| | Sample | Added amount of sample %(W/V) | Element | Measured value (ppm) |
|---|---|---|---|---|
| Ex. 21 | regenerated collagen powder | 1.00 | P | 17 |
| Comp. Ex. 15 | Allophane | 1.00 | P | 23 |

### (Example 22, Comparative Example 16)

The relationship between particle size and antibacterial activity level was examined. The regenerated collagen powder having an average particle size of 8.8, 63 and 1000 microns was added in an amount of 0, 2.5, 5, 7.5 and 10 (mg/ml) to liquid culture mediums (L-broth available from Difco Inc., yeast extract: 0.5%, bactopeptane: 1%, NaCI: 0.5%). Escherichia coli (E. coli IFO3972) as a test strain was inoculated into the mediums, and incubated with shaking at 37°C overnight. After that, the presence/absence of growth was determined visually.

As Example 22, a regenerated collagen powder having an average particle size of 8.8 microns and a regenerated collagen powder having an average particle size of 63 microns were added to liquid culture mediums (L-broth available from Difco Inc., yeast extract: 0.5%, bactopeptone: 1%, NaCl: 0.5%) such that the amount would be 7.5 and 10 (mg/ml). Escherichia coli (E. coli IFO3972) as a test strain was inoculated into the media, and incubated with shaking at 37°C overnight. After that, the presence/absence of growth was determined visually.

In Comparative Example 16, the test was performed in the same manner as in Example 22, except that the regenerated collagen powders were not added but regenerated collagen having an average particle size of 8.8 and 63 microns was added such that the amount would be 2.5 and 5 (mg/ml), and the regenerated collagen powder having an average particle size of 1000 microns was added such that the amount would be 2.5, 5, 7.5 and 10 (mg/ml).

The results of Example 22 and Comparative Example 16 are shown in Table 15.

**[Table 15]**

| | Added concentration of regenerated collagen powder (mg/ml) | | | | | | |
|---|---|---|---|---|---|---|---|
| | Comparative Example 16 | | | | | Example 22 | |
| Average particle size (µm) | 0 | 2.5 | 5 | 7.5 | 10 | 7.5 | 10 |
| 8.8 | C | C | C | - | - | A | A |
| 63 | C | C | C | - | - | C | A |
| 1000 | C | C | C | C | C | - | - |

Note: the criteria for growth evaluation were: A: No growth, B: Slight growth, and C: Growth observed. The same applies to the tables given below.

### (Example 23, Comparative Example 17)

The antibacterial property evaluation was performed for an insolubilized carboxymethyl cellulose (CMC) powder. As Example 23, the powder obtained in Production Example 10 was added to a liquid culture medium (L-broth available from Difco Inc., yeast extract: 0.5%, bactopeptone: 1%, NaCl: 0.5%) such that the amount would be 10 and 100 (mg/ml). Escherichia coli (E. coli IFO3972) as a test strain was inoculated into the medium, and incubated with shaking at 37°C overnight. After that, the presence/absence of growth was determined visually.

In Comparative Example 17, the test was performed in the same manner as in Example 23, except that the powder was not added to the liquid culture medium, but an insolubilized CMC powder was added in an amount of 5 mg/ml, and that untreated CMC was added in an amount of 5 and 10 (mg/ml).

The results of Example 23 and Comparative Example 17 are shown in Table 16.

**[Table 16]**

| | Comparative Example 17 | | | Example 23 | |
|---|---|---|---|---|---|
| Added concentration of insolubilized CMC powder (mg/ml) | 0 | 0 | 0 | 10 | 100 |
| Added concentration of non-processed CMC powder (mg/ml) | 5 | 10 | 0 | 0 | 0 |
| Growth evaluation | C | C | C | A | A |

### (Example 24, Comparative Example 18)

The antibacterial property evaluation was performed for an insolubilized polyvinyl alcohol (PVA) powder. As Example 24, the powder of Production Example 11 was added to a liquid culture medium (L-broth available from Difco Inc., yeast extract: 0.5%, bactopeptone: 1%, NaCl: 0.5%) such that the amount would be 10 (mg/ml). Escherichia coli (E. coli IFO3972) as a test strain was inoculated into the medium, and incubated with shaking at 37°C overnight. After that, the presence/absence of growth was determined visually.

In Comparative Example 18, the test was performed in the same manner as in Example 24, except that the powder was not added to the liquid culture medium, but an insolubilized A-17 powder was added such that the amount would be 5 mg/ml, and that untreated AF-17 was added such that the amount would be 5 and 10 (mg/ml).

The results of Example 24 and Comparative Example 18 are shown in Table 17.

**[Table 17]**

| | Comparative Example 18 | Example 24 |
|---|---|---|
| Added concentration of insolubilized PVA powder (mg/ml) | 0 | 10 |
| Added concentration of non-processed PVA powder (mg/ml) | 5 | 0 |
| Growth evaluation | C | A |

As described above, it was confirmed that the aluminum salt-containing resin powder of the present invention has a high phosphorus adsorption capability, and thus it adsorbs elemental phosphorus or a phosphorus compound. It also was confirmed that the aluminum salt-containing resin powder of the present invention has a high phosphorus adsorption capability, and thus it traps phosphorus, which is a nutrient for bacteria, and exhibits an antibacterial property and an antifungal property.

## Claims

1. An aluminum salt-containing resin powder comprising at least one matrix resin component selected from regenerated collagen, polyvinyl alcohol and carboxymethyl cellulose and an aluminum salt,
wherein the aluminum salt is chemically bonded to the matrix resin component, and the resultant is powdered.

2. The aluminum salt-containing resin powder according to claim 1,
wherein the matrix resin is cross-linked with the aluminum salt.

3. The aluminum salt-containing resin powder according to claim 1 or 2,
wherein the matrix resin further includes a cross-linking component made of an organic compound.

4. The aluminum salt-containing resin powder according to claim 3,
wherein the organic compound is a mono-functional epoxy compound represented by the following general formula (1), 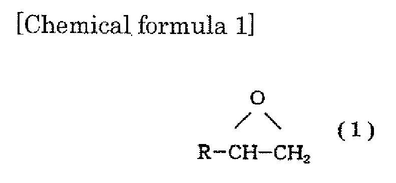 where R represents a substituent represented by R₁-, R₂-O-CH₂- or R₂-COO-CH₂-, R₁ represents a hydrocarbon group having a carbon number of 2 or more, or CH₂Cl, and R₂ represents a hydrocarbon group having a carbon number of 4 or more.

5. The aluminum salt-containing resin powder according to any one of claims 1 to 4, wherein the aluminum salt is a basic aluminum chloride or basic aluminum sulfate represented by the following formula:
Al(OH)ₙCl₃₋ₙ, or Al₂(OH)₂ₙ(SO₄)₃₋ₙ,
where n is 0.5 to 2.5

6. The aluminum salt-containing resin powder according to any one of claims 1 to 5, wherein the aluminum salt-containing resin powder has an average particle size of 0.01 to 80 µm.

7. The aluminum salt-containing resin powder according to any one of claims 1 to 6, wherein the content of aluminum in the aluminum salt-containing resin powder is within a range ranging from 0.1 to 70 wt% on a metallic simple substance basis.

8. The aluminum salt-containing resin powder according to any one of claims 1 to 7, wherein the aluminum salt-containing resin powder has a phosphorus adsorption capability.

9. The aluminum salt-containing resin powder according to any one of claims 1 to 7, wherein the aluminum salt-containing resin powder has an antibacterial property.

10. The aluminum salt-containing resin powder according to any one of claims 1 to 7, wherein the aluminum salt-containing resin powder has an antifungal property.

11. A resin composition comprising 0.1 wt% or more and 80 wt% or less of the aluminum salt-containing resin powder according to any one of claims 1 to 10 and 20 wt% or more and 99.9 wt% or less of a resin other than the aluminum salt-containing resin.

12. A process for producing an aluminum salt-containing resin powder comprising the steps of:
bringing an aluminum salt into contact with at least one water-soluble matrix resin gel component selected from regenerated collagen, polyvinyl alcohol and carboxymethyl cellulose so as to chemically bond the aluminum salt to the matrix resin gel component to obtain a water insoluble resin;
drying the water-insoluble resin; and
pulverizing the dried water insoluble resin into powder.

13. The process for producing an aluminum salt-containing resin powder according to claim 12, wherein, in the step of obtaining a water insoluble resin, the water-soluble matrix resin gel component is extruded into a fiber form or film form through a nozzle, and is brought into contact with an aluminum salt-containing aqueous solution to obtain a water insoluble resin.

14. The process for producing an aluminum salt-containing resin powder according to claim 12, wherein, in the powdering step, a fiber or film that has been cross-linked with the aluminum salt is cut and/or pulverized.

15. The process for producing an aluminum salt-containing resin powder according to claim 12, wherein, in the step of obtaining a water insoluble resin, an aluminum salt-containing aqueous solution is dripped onto the water-soluble matrix resin gel component to obtain a water insoluble resin.

16. The process for producing an aluminum salt-containing resin powder according to any one of claims 12 to 15, wherein the aluminum salt-containing resin powder has an average particle size of 0.01 to 80 µm.

17. The process for producing an aluminum salt-containing resin powder according to any one of claims 12 to 16, wherein the content of aluminum in the aluminum salt-containing resin powder is within a range ranging from 0.1 to 70 wt% on a metallic simple substance basis.

18. A phosphorus adsorbent comprising the aluminum salt-containing resin powder according to any one of claims 1 to 10 or the resin composition according to claim 11.

19. An antibacterial agent comprising the aluminum salt-containing resin powder according to any one of claims 1 to 10 or the resin composition according to claim 11.

20. An antifungal agent comprising the aluminum salt-containing resin powder according to any one of claims 1 to 10 or the resin composition according to claim 11.
